# EUROPEAN PATENT SPECIFICATION

(11) **EP 4 004 550 B1**
(45) Date of publication and mention of the grant of the patent: **10.01.2024**
(21) Application number: 20750248.5
(22) Date of filing: 30.07.2020
(51) Int. Cl.: G01N 33/574, A61K 39/00

(54) **ANALYTICAL METHOD AND IMMUNOLOGICAL TREATMENT FOR BLADDER CANCER**
ANALYTISCHES VERFAHREN UND IMMUNOLOGISCHE BEHANDLUNG FÜR BLASENKREBS
PROCÉDÉ ANALYTIQUE ET TRAITEMENT IMMUNOLOGIQUE POUR LE CANCER DE LA VESSIE

(30) Priority: 30.07.2019 EP 19189239
(43) Date of publication of application: 01.06.2022
(73) Proprietor: Medizinische Hochschule Hannover, 30625 Hannover (DE)
(72) Inventor: BÜTTNER, Falk, 30625 Hannover (DE); ROSSDAM, Charlotte, 30625 Hannover (DE); GERARDY-SCHAHN, Rita, 30625 Hannover (DE); OBERBECK, Astrid, 30625 Hannover (DE); TEZVAL, Hossein, 30625 Hannover (DE)
(74) Representative: Taruttis, Stefan Georg
(86) International application number: PCT/EP2020/071613
(87) International publication number: WO 2021/019061

(56) References cited:
- US-A- 4 757 003
- CHIKARA OHYAMA: "Glycosylation in bladder cancer", INTERNATIONAL JOURNAL OF CLINICAL ONCOLOGY, vol. 13, no. 4, 1 August 2008 (2008-08-01) , pages 308-313, XP055571906, GB ISSN: 1341-9625, DOI: 10.1007/s10147-008-0809-8 cited in the application
- DINGHAO ZHUO ET AL: "Biological Roles of Aberrantly Expressed Glycosphingolipids and Related Enzymes in Human Cancer Development and Progression", FRONTIERS IN PHYSIOLOGY, vol. 9, 3 May 2018 (2018-05-03), XP055622783, DOI: 10.3389/fphys.2018.00466
- ELINE OEYEN ET AL: "Bladder Cancer Diagnosis and Follow-Up: The Current Status and Possible Role of Extracellular Vesicles", INTERNATIONAL JOURNAL OF MOLECULAR SCIENCES, vol. 20, no. 4, 14 February 2019 (2019-02-14), page 821, XP055622789, DOI: 10.3390/ijms20040821
- SHAHIDUL ALAM ET AL: "Altered (neo-) lacto series glycolipid biosynthesis impairs [alpha]2-6 sialylation on N-glycoproteins in ovarian cancer cells", SCIENTIFIC REPORTS, vol. 7, no. 1, 30 March 2017 (2017-03-30), XP055729847, DOI: 10.1038/srep45367
- KATSUMI TSUKAZAKI ET AL: "Abnormal Expression of Blood Group-related Antigens in Uterine Endometrial Cancers", JAPANESE JOURNAL OF CANCER RESEARCH, vol. 82, no. 8, 1 August 1991 (1991-08-01) , pages 934-941, XP055729857, JP ISSN: 0910-5050, DOI: 10.1111/j.1349-7006.1991.tb01924.x
- Y. FUKUSHI ET AL: "Expression of Lacto Series Type 2 Antigens in Human Renal Cell Carcinoma and Its Clinical Significance", JOURNAL OF THE NATIONAL CANCER INSTITUTE, vol. 81, no. 5, 1 March 1989 (1989-03-01), pages 352-358, XP055729888, GB ISSN: 0027-8874, DOI: 10.1093/jnci/81.5.352

## Description

The present invention relates to an analytical method for detecting bladder cancer, by analysing a urine sample for presence of at least one specific tumour antigen. Further, the invention relates to compounds for use in the treatment of bladder cancer, e.g. for use in a method of treatment of bladder cancer, wherein the compounds are specific for the tumour antigen. In this embodiment, the compound can e.g. be an antibody specific for the tumour antigen.

The tumour antigen provided by the invention has the advantage that its detection in a urine sample is specific for the presence of bladder cancer. Optionally, the tumour antigen is analysed in combination with a further tumour antigen which has been found to also indicate presence of bladder cancer, and/or is analysed in combination with one or two further analytes for which elevated levels in urine have been found to indicate bladder cancer.

The invention also relates to an immunological treatment bladder cancer by providing a peptide binding to the tumour antigen for use in the treatment of bladder cancer. The peptide binding to the tumour antigen can e.g. be an antibody or be a part of a chimeric antigen receptor (CAR) for expression in an immune cell. The peptide binding to the tumour antigen may be an immunoglobulin, e.g. IgG, IgM, IgA, or IgG, or an antigen binding fragment thereof, or an scFv.

Further, invention relates to compounds for use in the treatment of bladder cancer, wherein the compounds contain or consist of the at least one specific tumour antigen, e.g. the at least one tumour antigen for use in the treatment of bladder cancer, e.g. the tumour antigen as a vaccine for use in the treatment of or prevention of bladder cancer.

### State of the art

Deb and Kumar, Journal of Cancer Research and Treatment, 34-38 (2018) recite several proteinaceous molecules and micro RNAs that had been identified as possible indicators of bladder cancer found in urine, and conclude that tumour heterogeneity would not allow for use of a single biomarker.

Azevedo et al., Oncotarget 91734-91764 (2017) review glycoproteins, glycolipids and proteoglycans that have been found in bladder cancer.

Ohyama, Int J Clin Oncol 308-313 (2008) reviews carbohydrate synthesis by normal and tumour cells and the role of glycoproteins, proteoglycans and glycosphingolipids in cellular interactions, quoting that the blood group carbohydrate antigen Lewis X could be shown to be present in up to 90% of bladder cancer tumours, e.g. on exfoliated cells of urine samples, by a monoclonal anti-Lewis X antibody.

WO 2007/023191 A2 describes 836 peptides identifiable by retention times in capillary electrophoresis as markers for bladder cancer.

Rossdam et al., Anal. Chem. 2019, 91, 6413-6418, describe an analytical method for the analysis of glycosphingolipid glycosylation by multiplexed capillary gel electrophoresis coupled to laser-induced fluorescence detection (xCGE-LIF) for the identification of cell surface markers of human induced pluripotent stem cells and derived cardiomyocytes.

### Object of the invention

It is an object of the invention to provide a tumour-specific antigen that is suitable for analysis in a urine sample and indicates presence, respectively absence, of bladder cancer. A further object is to provide a new therapeutic target specific for bladder cancer, and to provide a compound for use in the treatment of bladder cancer.

### Description of the invention

The invention achieves the object by the features of the claims, especially by providing an analytical method for detecting bladder cancer by analysing a urine sample for presence of a tumour antigen, the presence of which has been identified to indicate bladder cancer. The analytical method has the advantage that the tumour antigen can be analysed in a urine sample and has a high accuracy for indicating presence of bladder cancer, e.g. the method has a low rate of false positive results. According to the invention, the tumour antigen is the glycan consisting of lacto-N-neo-tetraose (Galβ1-4GlcNAcβ1-3Galβ1-4Glc, nLc4), which originates from the glycosphingolipid neolactotetraosyl ceramide and which was found to be highly elevated in urine samples of patients with bladder cancer compared to urine samples of persons free from bladder cancer. Optionally, nLc4 is analysed in combination with lactose (Galβ1-4Glc) as an additional tumour antigen, which originates from the glycosphingolipid lactosyl ceramide and which has been identified to be augmented in urine samples of patients with bladder cancer as well. Further additionally, nLc4 and lactose are analysed in combination with globotriaose (Galα1-4Galβ1-4Glc, Gb3) as an additional tumour antigen, which originates from the glycosphingolipid globotriaosyl ceramide and which has been identified to be augmented in urine samples of patients with bladder cancer as well. The glycans globotetraose (GalNAcβ1-3Galα1-4Galβ1-4Glc, Gb4), originating from the glycosphingolipids globotetraosyl ceramide was not significantly different between the group with bladder cancer and the control group and is optionally used as an internal standard, e.g. as a reference glycan. Accordingly, high concentrations of nLc4, optionally in combination with high concentrations of lactose and/or of Gb3 indicate presence of bladder cancer, whereas low concentrations of nLc4, of lactose and/or of Gb3 indicate absence of bladder cancer. The bladder cancer positive indicators nLc4, lactose and Gb3 originate from glycosphingolipids. In the analytical method, the presence of the glycan consisting of lacto-N-neo-tetraose (nLc4), which preferably originates from neolactotetraosyl ceramide, was found to be indicative of bladder cancer. In contrast, elongated glycosphingolipid glycans that are derived from nLc4, e.g. glycans having a longer chain of sugar moieties than nLc4, e.g. fucosyl-lacto-N-neo-tetraose or sialyl-lacto-N-neo-tetraose having an additional fucose or sialic acid attached to the structure of nLc4, respectively, have been found not to be indicative of bladder cancer. Accordingly, glycans having additional sugar moieties attached to the structure of lacto-N-neo-tetraose are preferably excluded from the present invention, e.g. excluded from the antigens to be analysed, and excluded from the antigen for which a binding peptide has specificity, e.g. against which an antibody or a CAR is directed.

In the invention, the glycan consisting of nLc4, optionally additionally the glycan consisting of lactose, optionally additionally the glycan consisting of Gb3 and/or Gb4, can be determined as the free glycan consisting of nLc4, of lactose, respectively of Gb3 and/or Gb4, or, alternatively, these glycans can be determined as their respective glycosphingolipids, i.e. as neolactotetraosyl ceramide, lactosyl ceramide, globotriaosyl ceramide, and/or globotetraosyl ceramide, respectively.

In the analytical method, the free glycans nLc4, lactose, Gb3, and/or Gb4 can be determined after hydrolysis of the respective glycosphingolipids, i.e. after hydrolysis of neolactotetraosyl ceramide, of lactosyl ceramide, of globotriaosyl ceramide and/or globotetraosyl ceramide, e.g. by chemical or enzymatic hydrolysis with ceramide glycanases or endoglycoceramidases directly in the urine sample or, preferably, by hydrolysis of the lipid fraction generated, e.g. isolated, from the urine sample. Alternatively, the respective glycosphingolipids neolactotetraosyl ceramide, lactosyl ceramide, globotriaosyl ceramide, and/or globotetraosyl ceramide can be determined directly in the urine sample or, preferably, in the lipid fraction isolated from the urine sample.

A lipid fraction can be generated from the urine sample e.g. by adsorption to a lipophilic adsorbent, preferably by extraction of the urine sample with organic solvents, and separating the lipophilic phase from the aqueous phase, optionally with removing a lipophilic adsorbent, respectively removing the organic solvents.

Optionally, in the analytical method, a membrane fraction is prepared from the urine sample, from which a lipid fraction can be prepared, e.g. by adsorption of the membrane fraction to a lipophilic adsorbent, preferably by extraction of the membrane fraction with organic solvents, preferably with subsequently separating the lipophilic adsorbent, respectively removing the organic solvents. The membrane fraction can be prepared by concentrating and separating the cells and/or membrane vesicles from the urine sample, e.g. by centrifugation or filtration, and removing the cell-free and/or the membrane vesicle-free fraction. Optionally, a fraction containing essentially only membrane vesicles is isolated from the urine sample, which fraction preferably is cell-free, e.g. by firstly removing cells, optionally also cell-debris, from the urine sample, e.g. by centrifugation or by filtration, and secondly concentrating membrane vesicles from the remaining liquid phase, e.g. by ultracentrifugation or by membrane filtration.

Optionally, a detergent may be added to the urine sample or a membrane fraction thereof, or a lipid fraction thereof, in order to solubilize neolactotetraosyl ceramide, lactosyl ceramide, globotriaosyl ceramide and/or globotetraosyl ceramide, optionally with subjecting the mixture with the detergent to agitation and/or to ultrasound.

An elevated molar concentration of the glycan consisting of nLc4, respectively of its ceramide neolactotetraosyl ceramide, optionally additionally an elevated molar concentration of lactose, respectively of lactosyl ceramide, optionally additionally an elevated molar concentration of Gb3, respectively of globotriaosyl ceramide indicates presence of bladder cancer. The elevated molar concentration can be determined in comparison to the concentration, preferably to the mean concentration, determined from urine samples from healthy persons. For example, an elevated molar concentration of nLc4 is higher by a factor of at least 1.5, a factor of at least 2, a factor of at least 3, a factor of at least 4, a factor of at least 5, a factor of at least 6 or a factor of at least 7 or of at least 10 or at least 15 or at least 20 than the mean concentration of nLc4 determined from the urine of healthy persons.

Advantageously, it was found that the molar concentrations of nLc4, of lactose, of Gb3, and of Gb4 in a healthy person are essentially independent from the age of the person. Further it was found that the molar concentrations of nLc4, of lactose, of Gb3, and of Gb4 in urine samples originating from patients diagnosed with bladder cancer were essentially independent from the ages of the patients.

Preferably, the molar concentration is determined in comparison to an internal standard. To allow quantitative determination of peak intensities, 0.083 ng of APTS-labeled Glyko^{®} Oligomannose 6 (Man6, available from Prozyme) was spiked in to each sample before xCGE-LIF measurement as an internal standard. The N-glycan Man6 is larger and more branched than glycosphingolipid (GSL)-derived glycans and therefore migrates more slowly through the capillary resulting in a higher migration time of approx. 280 migration time units (MTU). At this position, the obtained peak for Man6 does not interfere with any of the GSL-derived glycan peaks of the samples. Within a single measurement, the intensity of the Man6 peak was adjusted to 1 and the other peaks were adjusted accordingly based on their relative intensity compared to the Man6 peak. Intensities of peaks adjusted to the internal standard Man6 could then be quantitatively compared even between different measurements. Accordingly, preferably the measured intensities of the glycans that are measured are normalized to the intensity measured for Man6.

Alternatively, the molar concentration is determined as the relative molar concentration, i.e. as the proportion of the total molar concentration of glycans analysed, e.g. the total molar concentration of glycosphingolipids, or of free glycans of glycosphingolipids. Optionally, the molar concentration is determined in relation to the molar concentration of a protein and/or metabolite, e.g. albumin and/or creatinine, respectively, which protein or metabolite is present in urine. Preferably, the protein or metabolite has a concentration in urine which is not altered in patients with bladder cancer compared to persons without bladder cancer.

An elevated molar concentration of nLc4, or of neolactotetraosyl ceramide, optionally additionally an elevated molar concentration of lactose, respectively of lactosyl ceramide, optionally additionally an elevated molar concentration of Gb3, respectively of globotriaosyl ceramide, indicating presence of bladder cancer, can be determined by a peak intensity for the respective glycan which is higher by a factor of at least 1, a factor of at least 2, a factor of at least 3, a factor of at least 4, a factor of at least 5, a factor of at least 6 or a factor of at least 7 or of at least 10 or at least 15 or at least 20 of the intensity of the internal standard Man6 in a urine sample, preferably in a membrane vesicle fraction of a urine sample. Therein, the peak intensities are preferably normalized to the peak intensity of Man6.

Alternatively, an elevated molar concentration of nLc4, or of neolactotetraosyl ceramide, optionally additionally an elevated molar concentration of lactose, respectively of lactosyl ceramide, optionally additionally an elevated molar concentration of Gb3, respectively of globotriaosyl ceramide, indicating presence of bladder cancer, can be determined as at least 1%, at least 2%, at least 3%, at least 4%, at least 5%, at least 6%, at least 7%, at least 8%, at least 9%, e.g. at least 10%, of the total signal intensities measured for glycans, respectively for glycosphingolipids, in a urine sample, preferably in a membrane vesicle fraction of a urine sample.

The molar concentration of nLc4, optionally additionally of lactose, optionally additionally of Gb3, and/or Gb4 and/or of their respective glycosphingolipids can be determined by capillary gel electrophoresis coupled to multiplexed laser-induced fluorescence detection (xCGE-LIF), by chromatography, e.g. coupled to a mass spectrometer or based on fluorescence detection, or by mass spectrometry. In the analysis, especially for capillary gel electrophoresis, it is preferred that the sample is reacted with a marker that labels each glycan in a pre-determined quantitative manner, e.g. that labels only the reducing end of the glycan by a pre-determined number of marker molecules, e.g. that labels the reducing end of each glycan by 1 marker molecule. An exemplary marker is the fluorescent dye 8-aminopyrene-1,3,6-trisulfonic acid (APTS), which labels each reducing end of a glycan with 1 dye molecule. Glycan analysis by xCGE-LIF depends on determination of glycan-specific migration time units (MTUs). Glycan-specific migration times can be pre-determined once by analysis of the glycans, e.g. available as glycan standards, including nLc4, lactose, Gb3 and Gb4. Further preferred, in xCGE-LIF, an internal standard labelled with a different dye is added to the glycans prior to analysis by xCGE-LIF, and the MTUs of the internal standard are used for standardizing the MTUs of the glycans.

The analytical method preferably includes the step of transmitting the result of analysing the sample for the presence of the glycan to the patient from whom the urine sample originated. The method accordingly preferably comprises a step of transmitting the result of the analytical method to the person who is the origin of the urine sample, e.g. transmitting the result of the analytical method to the patient suspected of having a cancer of the urinary tract or to a person acting on behalf of the patient, e.g. to medical personnel.

The molar concentration of nLc4, optionally additionally the molar concentration of lactose, optionally additionally the molar concentration of Gb3, and/or the molar concentration of Gb4, as well as the respective glycosphingolipids from which these glycans originate, can be alternatively determined immunologically, using a peptide having binding specificity to nLc4, optionally additionally using a peptide having binding specificity to lactose, optionally additionally using a peptide having binding specificity to Gb3 and/or a peptide having binding specificity to Gb4. Preferably, the peptide having binding specificity to one of nLc4, lactose, Gb3, and Gb4 is an antibody or a lectin.

Generally, processes for generating an antibody having specificity for a specific antigen, herein the glycan consisting of nLc4, optionally additionally for the antigen consisting of lactose, optionally additionally for the glycan consisting of Gb3 and/or consisting of Gb4 are known, e.g. the hybridoma technology comprising fusing a tumour cell line with spleen cells of an animal experimentally vaccinated with the antigen, with subsequent screening of antibody producing hybridoma cells for secretion of a specific antibody. Further, the antigen binding section of an antibody can be generated by phage display methods and panning.

In a further embodiment, the invention also relates to nLc4, nLc4-ceramide or a nLc4-protein conjugate or a nLc4-derivative for use in the treatment of cancer of the urinary tract and/or in the urogenital tract.

In a further embodiment, the invention provides for a peptide, which preferably is an antibody or a CAR (chimeric antigen receptor, preferably expressed by a T-cell), which peptide specifically binds to nLc4, for use in the treatment of bladder cancer. The antibody or the antigen-binding domain of a CAR, specifically binding to nLc4, can be a natural antibody configuration, e.g. an IgG, preferably a humanized antibody, or a peptide comprising the paratope that specifically binds to nLc4, e.g. an scFv fragment, especially as an antigen-binding domain of a CAR. A CAR can e.g. comprise or consist of the antigen-binding domain that specifically binds to nLc4, a hinge, a transmembrane domain, at least one intracellular signalling domain, which comprises e.g. an intracellular hCD28 signalling domain and an intracellular hCD3zeta signalling domain.

The invention is now described by way of examples and with reference to the figures, which show in
- Fig. 1 electropherograms of urine samples of persons of the control group, and
- Fig. 2 electropherograms of urine samples of bladder cancer patients.
- Fig. 3 dot plots of relative signal intensities for a glycan consisting of nLc4 determined from urine samples of persons of the control group and in urine samples of bladder cancer patients,
- Fig. 4 dot plots of relative signal intensities for a glycan consisting of lactose determined from urine samples of persons of the control group and in urine samples of bladder cancer patients,
- Fig. 5 dot plots of relative signal intensities for a glycan consisting of Gb4 determined from urine samples of persons of the control group and in urine samples of bladder cancer patients,
- Fig. 6 dot plots of relative signal intensities for the glycan Gb3 determined from urine samples of persons of the control group and in urine samples of bladder cancer patients,
- Fig. 7 a comparison of relative signal intensities determined for glycans from urine samples of persons of the control group (left bars) and in urine samples of bladder cancer patients (right bars) for the MTUs indicated below and between the bars,

### Example 1: Detection of bladder cancer by xCGE-LIF analysis of glycans in urine samples

As an example for an analytical method, xCGE-LIF was used for the analysis of glycans that were obtained by hydrolysis of glycosphingolipids by ceramide glycanase of the membrane-vesicle fraction obtained from the cell-free liquid fraction of urine samples. In detail, urine samples, e.g. 40 mL, were centrifuged at 200 x g for 20 min at 4°C, and the supernatant was centrifuged at 2000 x g for 20 min at 4°C to prepare a liquid cell-free fraction. This cell-free fraction was ultra-centrifuged in a Beckman-Coulter Optima L-100K centrifuge at 12500 rpm (16039 x g) for 20 min at 4°C and filtrated through a 0.22 µm membrane filter for removing residual cell debris. The liquid cell-free urine fraction was then ultra-centrifuged in a Beckman-Coulter Optima L-100K centrifuge using a Type 70 Ti rotor at 34200 rpm (120066 x g) for 70 min at 4°C to pellet the membrane-vesicle fraction. The supernatant was removed, and the pellet containing the membrane vesicles was washed by resuspending in 5 mL PBS (phosphate buffered saline) and again ultra-centrifuged using a Type 70 Ti rotor at 34200 rpm (120066 x g) for 70 min at 4°C to pellet the membrane vesicle fraction again.

The pellet was resuspended in 1 mL 1:2 v/v chloroform/methanol, preferably by firstly adding the methanol portion and then adding the chloroform portion. Solubilisation was preferably supported by exposition to ultrasound. The solubilized chloroform/methanol mixture was centrifuged at 1620 x g for 5 min, and the supernatant was collected. The extraction of glycosphingolipids from the pellet was repeated twice using chloroform/methanol (2:1 (v/v)) and chloroform/methanol (1:1 (v/v)), respectively and the supernatants of the different extraction steps were pooled. The pooled supernatants were evaporated to dryness be removing the solvent, e.g. by a nitrogen stream. The dried glycolipids were dissolved in 1 mL methanol plus 2 mL water and then desalted applying on a Chromabond^{®} Cis ec polypropylene column that was pre-equilibrated with a sequence of chloroform, then 1:1 v/v chloroform/methanol, then methanol, then water. After applying the solubilized glycolipids to the pre-equilibrated column, the column was washed 3 x with water, then the glycolipids were eluted by methanol. The eluted glycolipids were again evaporated to dryness under nitrogen gas and stored at -20 °C until further use.

The eluted glycolipids were hydrolysed by ceramide glycanase derived from *Hirudo medicinalis* (GCase, obtained from Ludger, Oxfordshire, GB) in LudgerZyme ceramide glycanase reaction buffer for 24h at 37°C, or with endoglycoceramidase I (EGCase I, obtained from NewEngland Biolabs, Ipswich, USA) from *Rhodococcus triatomea* in the reaction buffer provided at 37°C for 16h. These enzymes cleave the β-glycosidic bond of glycosphingolipids and release the free glycan with a reducing end. For xCGE-LIF, the free glycans were fluorescence-labelled by APTS, obtained from Merck, Darmstadt, Germany.

The glycans were mixed with 2 µL 20 mM APTS in 3.5 M citric acid, 2 µL 2-picoline borane complex (2 M in DMSO, obtained from Merck) and 2 µl water and incubated for 16.5 h at 37°C in the dark. The reaction was stopped by adding 100 µL acetonitrile/water (80:20). Excess reagents were removed by hydrophilic liquid interaction chromatography applying Biogel P-10 gel (BioRad, Hercules, USA) in water/ethanol/acetonitrile (70:20:10) applied to the GHP membrane of a Pall Nanosep MF Centrifugal device (0.45 µm pore size, obtained from Merck). For pre-equilibration of the gel, solvent was removed by centrifugation at 54 x g for 1 min, and the gel was washed 3 x with water and then equilibrated with acetonitrile. Samples were applied to the suspended gel in 100 µL acetonitrile/water (80:20) with agitation for 5 min, liquid was removed by centrifugation at 54 x g for 1 min, discarding flow through, washing the gel 5 x with acetonitrile/water (80:20) containing 100 mM triethylamine (pH 8.5), then 3 x acetonitrile/water (80:20), wherein in each washing step the gel was shaken for 1 min at 450 rpm and centrifuged at 54 x g for 1 min. Elution of labelled glycans was done for three times with water by centrifugation of the gel upon incubation for 5 min at 450 rpm by shaking, respectively. The pooled eluates were then concentrated (SpeedVac) by evaporating the water in the dark and optionally stored at -20°C.

For xGCE-LIF, the resulting APTS-labelled glycans were suspended in water, e.g. 30 µL, LC-MS grade (Merck), and 3 µl of glycans were mixed with 1 µL GeneScan 500 LIZ dye size standard (DNA fragments of 20 to 500 bp for use in calibration of MTU, 1:50 dilution in Hi-Di Formamide (obtained from Applied Biosystems, Thermo Fisher Scientific, Foster City, CA)) and added up to 10 µL with Hi-Di formamide in a MicoAmp Optical 96-well reaction plate (obtained from Applied Biosystems, Thermo Fisher Scientific, Foster City, CA, USA). The mixture of ATPS-labelled glycans and the LIZ-labelled size standard was separated by multiplexed capillary gel electrophoresis (xCGE) on an ABI PRISM 3100-Avant Genetic Analyzer, remodeled by Axel Künzler, advanced biolab service GmbH, Munich, Germany.

The analyser was equipped with an array of 4 capillaries of 50 cm length that were filled with POP-7 polymer (obtainable from Applied Biosystems, Thermo Fisher Scientific). Running buffer was obtained from Applied Biosystems, Thermo Fisher Scientific. Samples were injected for 15 s at 1.6 kV, and after a data delay of 50 s, detection was for 30 min at 12 kV at 60°C using laser induced fluorescence detection and for analysis, the GeneMapper software v. 3.7 was used. For each measurement, an electropherogram was obtained displaying peak values for the size standard in one channel and for APTS-labelled glycans in another channel at specific migration time units (MTU) and with specific intensities (relative fluorescence units, RFU). A peak amplitude threshold of at least 10 RFU was set in the GeneMapper software for defining peaks.

For identification, known glycans were labelled with APTS and their MTUs were measured. Known glycans were used directly or after exoglycosidase digest using known enzymes. The following glycans were obtained and used as standards:

| product name | structure | supplier | name of glycan |
|---|---|---|---|
| Glyko^{®} Lacto-N-Fucopentaose III (LNFPIII) | Galβ1-4(Fucα1-3)GlcNAcβ1-3Galβ1-4Glc | Prozyme | Lewis^{r} pentaose |
| Glyko^{®} Lacto-N-Fucopentaose I (LNFPI) | Fucα1-2Galβ1-3GlcNAcβ1-3Galβ1-4Glc | Prozyme | fucosyl Lc4 / SSEA5 |
| Blood group H antigen pentaose type 2) Lacto-N-neofucapentaose I (LnNFPI) | Fucα1-2Galβ1-4GlcNAcβ1-3Galβ1-4Glc | Elicityl-OligoTech^{®} | fucosyl nLc4 |
| LS-Tetrasaccharide a (LSTa) | Neu5Acα2-3Galβ1-3GlcNAcβ1-3Galβ1-4Glc | Elicityl-OligoTech^{®} | sialyl Lc4 |
| SSEA-4 hexaose | Neu5Acα2-3Galβ1-3GalNAcβ1-3Galα1-Galβ1-4Glc | Elicityl-OligoTech^{®} | sialyl Gb5 / SSEA4 |
| Globo H | Fucα1-2Galβ1-3GalNAcβ1-3Galα1-4GaLβ1-4GLc | Elicityl-OligoTech^{®} | fucosyl Gb5 / globoH |
| Globotriaose (Gb3) | Galα1-4Galβ1-4Glc | Elicityl-OligoTech^{®} | Gb3 |
| Lacto-N-neotetraose (LNnT) | Galβ1-4GlcNAcβ1-3Galβ1-4Glc | Elicityl-OligoTech^{®} | nLc4 |
| Globopentaose (Gb5) | Galβ1-3GalNAcβ1-3Galα1-4Galβ1-4Glc | Elicityl-OligoTech^{®} | Gb5 / SSEA3 |
| Blood group A antigen hexaose type 1 | GalNAcα1-3(Fucα1-2)Galβ1-3GlcNAcβ1-3Galβ1-4Glc | Elicityl-OligoTech^{®} | A type 1 hexa |

Further, the following glycosphingolipids (GSL) were obtained and, 50 ng each, were hydrolysed and APTS-labelled as described above and used as standards:

| product name | structure | supplier | trivial name |
|---|---|---|---|
| Ganglioside GM3 | Neu5Acα2-3Galβ1-4Glcβ1Cer | Avanti^{®} | GM3 |
| Disialoganglioside GD₁ₐ | Neu5Acα2-3Galβ1-3GalNAcβ1-4(Neu5Acα2-3)Galβ1-4Glcβ1Cer | Merck | GD1a |
| Ganglioside GD₃ | Neu5Acα2-8Neu5Acα2-3Galβ1-4Glcβ1Cer | Merck | GD3 |
| Ganglioside GD_{1b} | Galβ1-3GalNAcβ1-4(Neu5Acα2-8Neu5Acα2-3)Galβ1-4Glcβ1Cer | Merck | GD1b |
| Ganglioside GD₂ | GalNAcβ1-4(Neu5Acα2-8Neu5Acα2-3)Galβ1-4Glcβ1Cer | Merck | GD2 |
| GM1 glycolipid | Galβ1-3GalNAcβ1-4(Neu5Acα2-3)Galβ1-Glcβ1Cer | Ludger | GM1 |

Avanti^{®} is available at Polar Lipids Inc.

As further standards, the known glycans, subsequent to being labelled with APTS, were digested with at least one of the following exoglycosidases:

| enzyme | supplier | dilution | incubation time |
|---|---|---|---|
| α(2-3,6,8)-Neuraminidase (250 mU/ml) | EY labs | 1:10 | 1 h |
| β(1-3)-Galactosidase (10,000 U/ml) | New England Biolabs^{®} | 1:10 | 3h |
| β{1-4,6)-Galactosidase (25 U/ml) | Prozyme | 3:10 | overnight |
| α(1-3,4,6)-Galactosidase (8,000 U/ml) | New England Biolabs^{®} | 1:10 | 3 h |
| α(1-2,3,4,6)-Fucosidase (2 U/ml) | Prozyme | 4:10 | 24 h |
| β-*N*-Acetyilhexosaminidase (5,000 U/ml) | New England Biolabs^{®} | 3:10 | over night |
| β-*N*-Acetylglucosaminidase (4,000 U/ml) | New England Biolabs^{®} | 3:10 | over night |
| α-*N*-Acetylgalactosaminidase (20,000 U/ml) | New England Biolabs^{®} | 1:10 | over night |

Non-specific activity of the exoglycosidases could be excluded by incubation with non-target glycans and subsequent analysis by xCGE-LIF.

The migration times ranging from 15 to 350 MTU measured could be analysed using CorelDRAW 2017. The intensity of the highest peak in each electropherogram was set to 1 for normalizing the relative fluorescence units. For quantitative analysis of peak intensities, the relative signal intensity of each peak was determined. Therefore, the heights of all peaks in the range from 15 to 300 MTU were summed up and the height of individual peaks was calculated as a percentage of the summed-up peak heights. For statistical analysis, the GraphPad Prism 4 software v. 4 was used. In figures, p-values of <0.05 upon unpaired Student's t-test were regarded as statistically significant and are depicted or highlighted by asterisks.

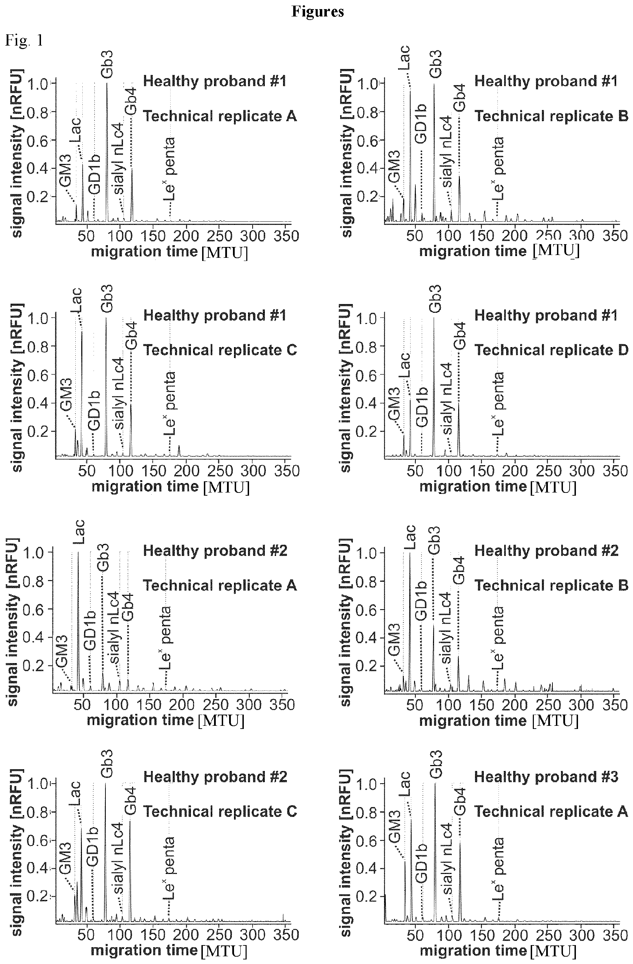

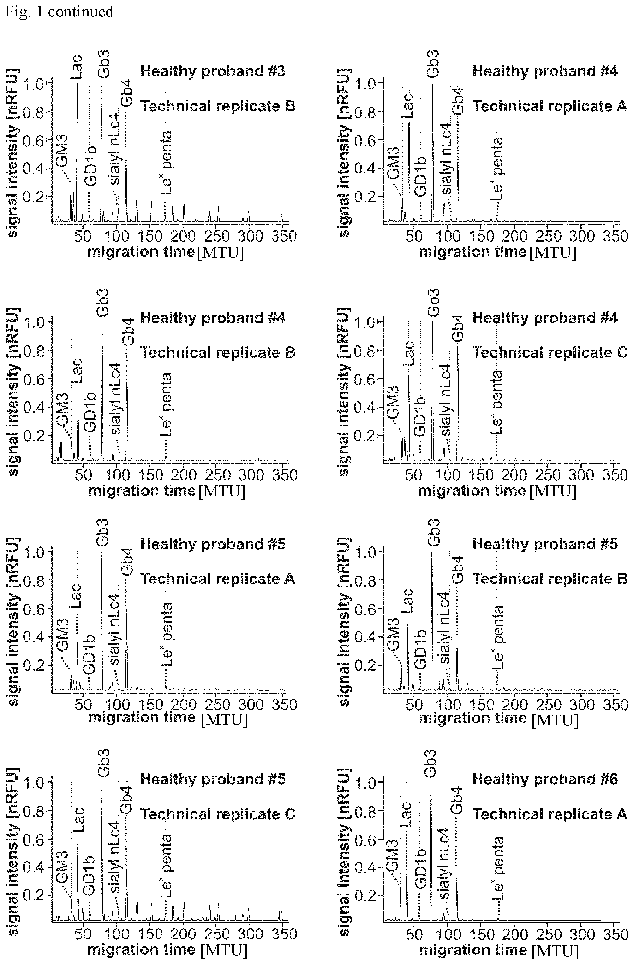

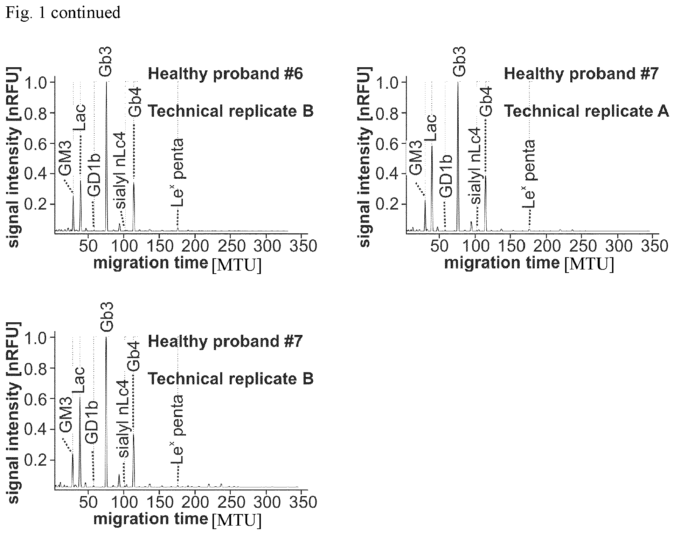

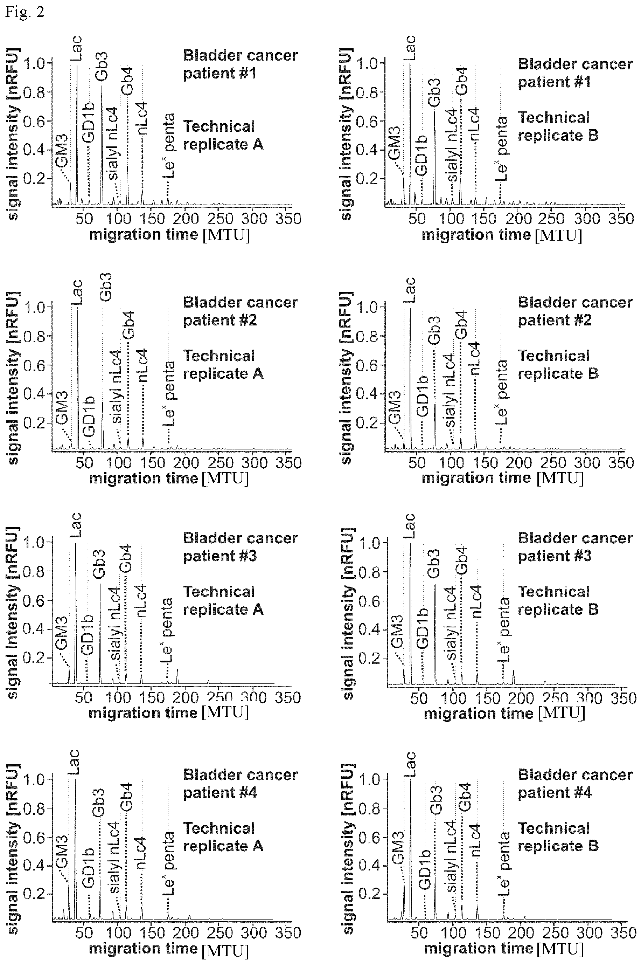

Fig. 1 shows electropherograms obtained by xCGE-LIF of the free glycans, labelled with APTS, obtained by enzymatic hydrolysis of the glycosphingolipids of membrane vesicles from urine samples of persons of the control group, and Fig. 2 shows electropherograms of urine samples of diagnosed bladder cancer patients. The glycans indicated were identified using the MTUs of known glycans. The results depicted in Fig. 1 and Fig. 2 show that in urine samples of persons of the control group, essentially no nLc4 could be detected in the glycosphingolipid fraction and that in urine samples of bladder cancer patients, nLc4 could be detected in the glycosphingolipid fraction.

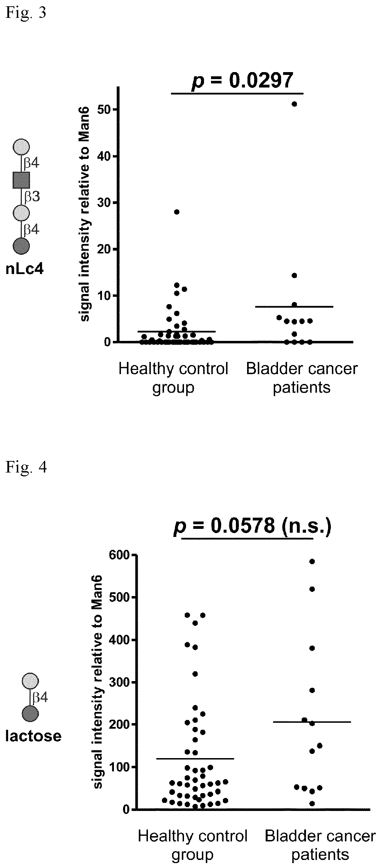

Fig. 3 shows signal intensities for nLc4 relative to the internal standard Man6 from 47 persons of the control group not suffering from bladder cancer (left) and from 13 bladder cancer patients (right). As the glycans are labelled at their reducing end with 1 marker molecule, the signal intensity directly corresponds to the relative molar concentration. It can be seen that the signal intensity for nLc4 is significantly higher in the group of patients with bladder cancer compared to the control group. The mean value of the signal intensity adjusted to Man6 is 7.6 for the patient group and 2.27 for the control group. The big difference in molar concentration of nLc4 in urine samples of bladder cancer patients shows that nLc4 is a highly specific indicator of bladder cancer, and that the very low or absent concentration of nLc4 in the urine samples of persons without bladder cancer avoids false positive indications. Further, the big difference in molar concentration of nLc4 in urine samples of bladder cancer patients allows the detection of nLc4 by a binding peptide having specificity for nLc4, e.g. an antibody specific for nLc4.

Fig. 4 shows signal intensities for lactose relative to the internal standard Man6 from 47 persons of the control group not suffering from bladder cancer (left) and from 13 bladder cancer patients (right). It can be seen that the signal intensity relative to the internal standard Man6 for lactose, which corresponds to the relative molar concentration, has a mean of about r 119 in samples from persons of the control group, and a mean of about 205 for samples from bladder cancer patients. n.s.: not significant.

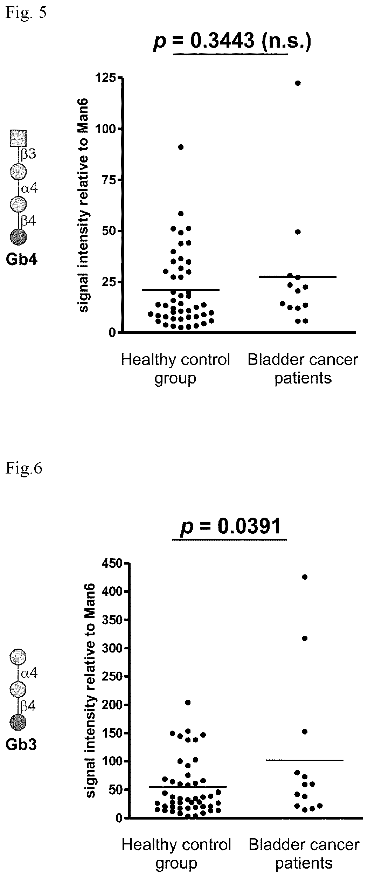

Fig. 5 shows signal intensities for Gb4 relative to the internal standard Man6 from 47 persons of the control group not suffering from bladder cancer (left) and from 13 bladder cancer patients (right). It can be seen that the signal intensity relative to the internal standard Man6 for Gb4, which corresponds to the relative molar concentration, has a mean of about 21 in samples from persons of the control group, and a mean of about 28 for samples from bladder cancer patients. n.s.: not significant.

Fig. 6 shows signal intensities for Gb3 relative to the internal standard Man6 from 47 persons of the control group not suffering from bladder cancer (left) and from 13 bladder cancer patients (right). It can be seen that the signal intensity relative to the internal standard Man6 for Gb3, which corresponds to the relative molar concentration, has a mean of about 54 in samples from persons of the control group, and a mean of about 102 for samples from bladder cancer patients.

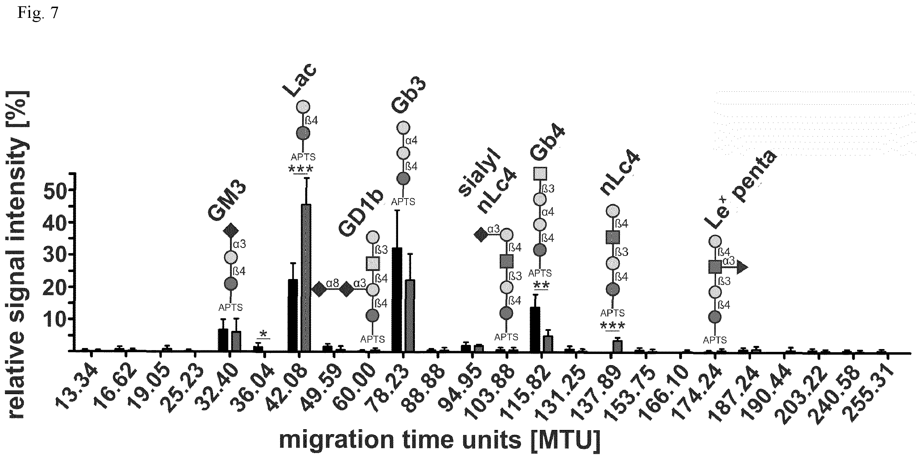

Fig. 7 shows MTUs for some glycans derived from the glycosphingolipids of the membrane vesicle fraction of urine samples of persons of the control group (left bar) and of bladder cancer patients (right bar), with the MTU values for the glycans indicated between and below the columns. It becomes clear that an elevated concentration of nLc4 and/or lactose is indicative of bladder cancer.

Table 1 shows individual signal intensities of the glycosphingolipid-derived glycans nLc4, lactose, Gb4 and Gb3 for all 60 persons analysed relative to the internal standard Man6. Based on the common medical examination, persons were grouped in the groups "bladder cancer" (13 persons, #1 to #13) or "control" (47 persons, #14 to #60) with the latter not suffering from bladder cancer. Based on calculation of sensitivity and specificity for nLc4 and lactose, cut-off values were defined (see Table 2) and values above the cut-off were highlighted in bold in this table. No signal for nLc4 was detected in the bladder cancer patients # 10-#13. However two of these patients display high values for the other positive marker lactose. It is known that cancers are genetically heterogeneous and it deserves further investigation of whether some types of bladder cancer do not produce nLc4 or whether the absence of nLc4 was caused by erroneously retrieval or storage of urine in the clinic.

Generally preferably, midstream morning urine is taken and directly frozen. Generally preferably, the concentration of the urine has to be determined by analysis of e.g. creatinine and the here postulated glycan markers should be related to the urine concentration. In addition to bladder cancer, other types of cancer associated with the urinary tract and/or in the urogenital tract, e.g. renal transitional cell carcinoma, malignant melanoma of the bladder or endometrial cancer showed values for nLc4 and/or lactose above the threshold. Cancers like prostate carcinoma or renal cell carcinoma mostly did not show apparent values for the analysed glycans.

**Table 1**

| **#** | **Group** | **Signal intensity relative to Man6** | | | | **Diagnosis of other types of cancer** |
|---|---|---|---|---|---|---|
| | | **nLc4** | **lactose** | **Gb4** | **Gb3** | |
| 1 | bladder cancer | **51.22** | **519.28** | 49.47 | 425.72 | |
| 2 | bladder cancer | **14.36** | **137.62** | 14.32 | 42.23 | |
| 3 | bladder cancer | **8.08** | **202.37** | 22.55 | 72.45 | |
| 4 | bladder cancer | **5.31** | **280.88** | 28.19 | 152.75 | renal transitional cell carcinoma |
| 5 | bladder cancer | **4.61** | **210.61** | 122.39 | 317.39 | |
| 6 | bladder cancer | **4.50** | 49.84 | 12.48 | 38.77 | |
| 7 | bladder cancer | **4.50** | 52.49 | 5.77 | 22.11 | |
| 8 | bladder cancer | **4.42** | 14.50 | 5.85 | 14.96 | renal pelvis cancer |
| 9 | bladder cancer | 1.76 | **150.12** | 23.53 | 58.76 | |
| 10 | bladder cancer | 0.00 | 50.95 | 27.24 | 79.95 | |
| 11 | bladder cancer | 0.00 | 43.00 | 20.60 | 59.60 | |
| 12 | bladder cancer | 0.00 | **584.74** | 13.53 | 21.72 | |
| 13 | bladder cancer | 0.00 | **380.50** | 12.10 | 17.10 | |
| 14 | control | 0.00 | 14.87 | 6.84 | 21.26 | prostate carcinoma |
| 15 | control | 0.00 | 9.54 | 3.18 | 8.72 | |
| 16 | control | 0.00 | 12.65 | 5.88 | 20.60 | |
| 17 | control | 0.00 | 33.46 | 13.39 | 32.86 | |
| 18 | control | 0.00 | 91.91 | 6.70 | 9.00 | gynaecological cancer |
| 19 | control | 0.00 | 17.42 | 7.71 | 19.50 | |
| 20 | control | 0.00 | 7.59 | 3.41 | 4.17 | prostate carcinoma |
| 21 | control | 0.00 | 33.31 | 13.69 | 27.69 | prostate carcinoma |
| 22 | control | 0.00 | 14.31 | 10.56 | 12.38 | |
| 23 | control | 0.00 | **457.50** | 27.38 | 28.44 | renal transitional cell carcinoma |
| 24 | control | 0.00 | **382.55** | 8.36 | 15.50 | |
| 25 | control | 0.00 | **440.00** | 27.45 | 45.78 | |
| 26 | control | 0.00 | **457.82** | 2.67 | 3.56 | |
| 27 | control | 0.00 | **225.05** | 5.60 | 13.41 | |
| 28 | control | 0.00 | 136.08 | 10.89 | 17.89 | renal cell carcinoma |
| 29 | control | 0.00 | **388.45** | 15.91 | 34.82 | prostate carcinoma |
| 30 | control | 0.00 | **210.33** | 9.81 | 27.10 | |
| 31 | control | 0.00 | **181.73** | 14.36 | 37.59 | |
| 32 | control | 0.00 | 62.26 | 3.85 | 21.29 | |
| 33 | control | 0.00 | 42.67 | 18.11 | 57.83 | |
| 34 | control | 0.00 | 56.06 | 12.06 | 28.24 | |
| 35 | control | 0.00 | 48.54 | 13.81 | 35.23 | renal cell carcinoma |
| 36 | control | 0.00 | 41.25 | 4.55 | 14.15 | |
| 37 | control | 0.00 | 12.39 | 7.96 | 28.98 | prostate carcinoma |
| 38 | control | 0.28 | 32.03 | 8.87 | 13.75 | |
| 39 | control | 0.34 | 57.35 | 36.34 | 61.00 | |
| 40 | control | 0.48 | 23.55 | 31.67 | 59.36 | |
| 41 | control | 0.60 | 21.47 | 20.01 | 37.78 | |
| 42 | control | 1.21 | 28.95 | 10.11 | 20.95 | |
| 43 | control | 1.29 | 21.88 | 7.88 | 39.29 | |
| 44 | control | 1.30 | 36.70 | 39.80 | 63.70 | |
| 45 | control | 1.31 | 74.27 | 2.85 | 17.92 | |
| 46 | control | 1.39 | 60.44 | 34.99 | 92.14 | |
| 47 | control | 1.52 | 69.09 | 19.78 | 75.26 | |
| 48 | control | 1.65 | 62.00 | 18.29 | 65.65 | renal cell carcinoma |
| 49 | control | 1.69 | 59.78 | 49.08 | 153.57 | |
| 50 | control | 2.27 | 98.33 | 34.77 | 100.23 | |
| 51 | control | 2.79 | 78.55 | 51.07 | 149.54 | |
| 52 | control | 3.48 | **204.38** | 51.24 | 138.10 | |
| 53 | control | 4.10 | 64.70 | 43.70 | 102.60 | |
| 54 | control | **4.97** | 92.45 | 44.03 | 138.00 | malignant melanoma grown into bladder |
| 55 | control | **6.22** | 134.04 | 30.22 | 68.30 | |
| 56 | control | **7.65** | **239.59** | 9.18 | 27.00 | |
| 57 | control | **10.54** | **163.82** | 29.96 | 146.86 | |
| 58 | control | **11.42** | 99.21 | 58.55 | 144.58 | |
| 59 | control | **12.28** | **188.41** | 12.62 | 44.28 | endometrial cancer |
| 60 | control | **28.00** | **319.76** | 91.08 | 204.32 | |

Table 2 shows determination of sensitivity and specificity for glycan intensities relative to the internal standard Man6 from urine samples of 60 persons of which 13 were diagnosed for bladder cancer and 47 did not suffer from bladder cancer. For the glycosphingolipid-derived glycan nLc4, a sensitivity of 62% and a specificity of 85% was obtained at a cut-off value of ≥4.42. For the glycosphingolipid-derived glycan lactose, a sensitivity of 62% and a specificity of 72% was obtained at a cut-off value of ≥137.62. A combined analysis of nLc4 and lactose led to the diagnosis "no bladder cancer" when both markers were below the above defined cut-off and led to the diagnosis "bladder cancer" if one of both markers was above the above defined cut-offs with a sensitivity of 85% and a specificity of 66%.

**Table 2**

| Marker | Intensity (cut-off) | Diagnosed as | Bladder cancer | Control | Sensitivity | Specificity |
|---|---|---|---|---|---|---|
| nLc4 | <4.42 | Negative | 5 | 40 | 0.62 | 0.85 |
| | ≥4.42 | Positive | 8 | 7 | | |
| lactose | <137.62 | Negative | 5 | 34 | 0.62 | 0.72 |
| | ≥137.62 | Positive | 8 | 13 | | |
| combined analysis of nLc4 and lactose | both values below cut-off | Negative | 2 | 31 | 0.85 | 0.66 |
| | at least one value above cut-off | Positive | 11 | 16 | | |

Table 3 shows statistical analyses for glycan intensities relative to the internal standard Man6 from urine samples of 60 persons of which 13 were diagnosed for bladder cancer and 47 did not suffer from bladder cancer by Student's t-test. The intensities for the glycosphingolipid-derived glycans nLc4 and Gb3 are significantly (p≤0.05) increased in the group with bladder cancer compared to the control group. For the marker lactose, the significance threshold was slightly failed.

**Table 3**

| **Marker** | **Bladder cancer** | | | **Control** | | | **p value t-test** |
|---|---|---|---|---|---|---|---|
| | **n** | **mean** | **std. dev** | **n** | **mean** | **std. dev** | |
| nLc4 | 13 | 7.60 | 13.72 | 47 | 2.27 | 4.92 | 0.0297 |
| lactose | 13 | 205.92 | 187.24 | 47 | 119.33 | 128.64 | 0.0578 |
| Gb4 | 13 | 27.54 | 30.75 | 47 | 21.07 | 18.57 | 0.3443 |
| Gb3 | 13 | 101.81 | 127.14 | 47 | 53.83 | 49.12 | 0.0391 |

### Example 2: Detection of bladder cancer by immunological analysis of nLc4 in urine samples

An antibody against nLc4, e.g. a commercially available antibody or an antibody derived from hybridoma cells generated from animals that were experimentally immunized with nLc4 can be used to detect nLc4 in the urine sample of patients with bladder cancer applying e.g. an ELISA (enzyme linked immuno sorbent assay) or in a RIA (radio immuno assay) or by immunochromatography, e.g. by lateral flow test. The antibody can be polyclonal or monoclonal. The sample could be the glycosphingolipid fraction isolated from a urine sample, with the β-glycosidic bond of glycosphingolipids optionally hydrolysed to release the free glycans. Alternatively, the sample could be the urine sample without pre-treatment, or the sample could be the urine sample, complete or with cells removed, optionally containing an added detergent, further optionally treated with ultrasound, to release glycosphingolipids from membranes. Alternatively or additionally, the urine sample, complete or with cells removed, could be treated by addition of a glycanase, e.g. a ceramide glycanase, to release the glycans from membrane-bound, respectively from solubilized, glycosphingolipids.

### Example 3: nLc4 for use in the immunotherapeutical treatment against bladder cancer.

In this embodiment, immunotherapy includes nLc4 for use in the treatment of bladder cancer. Patients suffering from bladder cancer can be administered (vaccinated) with a glycan comprising or consisting of nLc4 and/or nLc4-ceramide and/or a nLc4-protein conjugate and/or a nLc4-derivative in order to provoke a T-cell dependent or innate immune response against nLc4 in these patients that is then directed against the bladder cancer expressing the antigen epitope nLc4. The immune response can be supported by vaccination in combination with a T-cell carrier (e.g. keyhole limpet hemocyanin) or with adjuvants (e.g. mycobacterium cell-wall skeleton or QS-21 or saponin-based adjuvant).

### Example 4: Immunotherapy against bladder cancer by using an antibody specific for nLc4

In this embodiment, an antibody that is specific for nLc4 is for use in the treatment of bladder cancer, which antibody is directed against nLc4 as a molecular target.

A monoclonal or polyclonal antibody against nLc4, e.g. a commercially available antibody or an antibody derived from hybridoma cells generated from animals that were experimentally immunized with nLc4 can be applied for immunotherapy against bladder cancer. Accordingly, the antibody specific for nLc4 can be a human or a non-human antibody.

For immunotherapy, patients can be directly administered with the nLc4-specific (monoclonal) antibody. This antibody can then activate complement- or cellular-dependent anticancer activities. Such an antibody might be also linked to a cytotoxic agent, e.g. the antibody can be biotinylated and then linked a (strept)avidin-tagged cytotoxic protein, e.g. saporin, a ribosome-inactivating toxin or directly linked to a cytotoxic agent, e.g. ricin chain A toxin or linked to a cytokine.

### Example 5: CAR comprising protein binding of nLc4 for use in the treatment of bladder cancer

The paratope of an antibody binding to nLc4 is used to generate a respective single-chain variable fragment (scFv) binding to nLc4. This scFv was used as a portion of a CAR (chimeric antigen receptor), which from N-terminus to C-terminus contained the single-chain variable fragment antibody domain (scFv), a modified hCD8 hinge, a hCD8 transmembrane domain, an intracellular hCD28 signalling domain and an intracellular hCD3ζ (hCD3 zeta) signalling domain, which preferably are linked to one another from N-terminus to C-terminus, more preferably directly linked to one another form N-terminus to C-terminus. The CAR can contain a hΔFc IgG domain in the alternative to a modified hCD8 hinge, and/or a fusion hCD28 transmembrane domain - hCD28/CD3 zeta domain in the alternative to a hCD8 transmembrane domain, an intracellular hCD28 signalling domain and an intracellular hCD3ζ (hCD3 zeta) signalling domain.

A nucleic acid sequence encoding the CAR containing a scFv domain having specificity for nLc4 could be expressed in immune cells, e.g. T-cells, preferably in primary T-cells or NK cells, which cells could be introduced into an experimental animal having bladder cancer. The immune cells expressing the CAR reduced the presence of bladder cancer in the recipient.

## Claims

1. Analytical method for detecting cancer of the bladder , comprising analysing a urine sample for the presence of the glycan consisting of Galβ1-4GlcNAcβ1-3Galβ1-4Glc (lacto-N-neo-tetraose, neolactotetra, nLc4).

2. Analytical method according to claim 1, **characterized by** analysing for the presence of the glycan consisting of nLc4 by contacting the sample with a peptide having specificity for binding nLc4.

3. Analytical method according to one of the preceding claims, **characterized by** analysing for the presence of the glycan consisting of nLc4 by capillary gel electrophoresis of glycans obtained by hydrolysis of glycosphingolipids of a cell-free membrane vesicle fraction isolated from the urine sample.

4. Analytical method according to one of the preceding claims, **characterized in that** a molar concentration of at least 1-fold of the internal standard Oligomannose 6 (Man6) or of at least 1 % of nLc4 of the total of glycans originating from glycosphingolipids of the sample indicates presence of cancer of the bladder.

5. Analytical method according to claim 4, **characterized in that** the molar concentration is determined as the signal intensity.

6. Analytical method according to one of the preceding claims, **characterized by** additionally analysing the urine sample for presence of the glycan consisting of lactose.

7. Analytical method according to one of the preceding claims, **characterized by** additionally analysing the urine sample for presence of the glycan consisting of GalNAcβ1-3Galα1-4Galβ1-4Glc (globotetraose, Gb4).

8. Analytical method according to one of the preceding claims, **characterized by** additionally analysing the urine sample for presence of the glycan consisting of Galα1-4Galβ1-4Glc (globotriaose, Gb3).

9. Analytical method according to one of the preceding claims, **characterized in that** a membrane fraction is prepared from the urine sample, from which a lipid fraction is prepared, and analysing the lipid fraction for the presence of the glycan.

10. Analytical method according to one of the preceding claims, **characterized in that** the result of analysing the sample for the presence of the glycan is transmitted to the patient from whom the sample originated or to a person acting on behalf of the patient.

11. An antibody or a CAR expressed by a T-cell, or a portion thereof, having specificity for binding a glycan consisting of nLc4, for use in the treatment of bladder cancer .

12. Compound comprising or consisting of nLc4 and/or nLc4-ceramide and/or a nLc4-protein conjugate for use as a vaccine in the treatment or prevention of cancer of the bladder.

13. Compound for use in the treatment of cancer of the bladder according to claim 12, wherein the compound is comprised in a formulation suitable for intramuscular or for systemic administration.

## Patentansprüche

1. Analyseverfahren zum Nachweisen von Blasenkrebs, dass das Analysieren einer Urinprobe auf Gegenwart des Glycans umfasst, das aus (Galβ1-4GlcNAcβ1-3Galβ1-4Glc (lacto-N-neo-tetraose, neolactotetra, nLc4)) besteht.

2. Ein Analyseverfahren nach Anspruch 1, **gekennzeichnet durch** das Analysieren auf Gegenwart des Glycans, das aus nLc4 besteht, durch Kontaktieren der Probe mit einem Peptid, das die Spezifität zum Binden von nLc4 hat.

3. Analyseverfahren nach einem der voranstehenden Ansprüche, **gekennzeichnet durch** das Analysieren auf Gegenwart des Glycans, das aus nLc4 besteht, durch Kapillargelelektrophorese von Glycanen, die durch Hydrolisieren von Glycosphlingolipiden einer zellfreien Membranvesikelfraktion erhalten wurden, die aus einer Urinprobe isoliert wurde.

4. Analyseverfahren nach einem der voranstehenden Ansprüche, **dadurch gekennzeichnet, dass** eine molare Konzentration von wenigstens 1-fach des internen Standards Oligomannose 6 (Man6) oder von wenigstens 1 % von nLc4 der Gesamtheit von Glycanen, die aus Glycosphlingolipiden der Probe stammen, das Vorliegen von Blasenkrebs anzeigt.

5. Analyseverfahren nach Anspruch 4, **dadurch gekennzeichnet, dass** die molare Konzentration als die Signalintensität bestimmt wird.

6. Analyseverfahren nach einem der voranstehenden Ansprüche, **gekennzeichnet durch** zusätzliches Analysieren der Urinprobe auf Gegenwart des Glycans, das aus Laktose besteht.

7. Analyseverfahren nach einem der voranstehenden Ansprüche, **gekennzeichnet durch** zusätzliches Analysieren der Urinprobe auf Vorliegen des Glycans, das aus GalNAcβ1-3Galα1-4Galβ1-4Glc (Globotetrose, Gb4) besteht.

8. Analyseverfahren nach einem der voranstehenden Ansprüche, **gekennzeichnet durch** das zusätzliche Analysieren der Urinprobe auf Vorliegen des Glycans, das aus Galα1-4Galβ1-4Glc (Globotriaose, Gb3) besteht.

9. Analyseverfahren nach einem der voranstehenden Ansprüche, **dadurch gekennzeichnet, dass** eine Membranfraktion aus der Urinprobe prepariert wird, aus der eine Lipidfraktion präpariert wird, und Analysieren der Lipidfraktion auf das Vorliegen des Glycans.

10. Analyseverfahren nach einem der voranstehenden Ansprüche, **dadurch gekennzeichnet, dass** das Ergebnis des Analysierens der Probe auf Vorliegen des Glycans an den Patienten übermittelt wird, von dem die Probe stammt oder an eine Person, die im Auftrag des Patienten handelt.

11. Antikörper oder von einer T-Zelle exprimierter CAR oder ein Teil davon, mit Spezifität für das Binden eines Glycans, das aus nLc4 besteht, zur Verwendung bei der Behandlung von Blasenkrebs.

12. Verbindung, die nLc4 und/oder nLc4-Ceramit und/oder ein nLc4 -Proteinkonjugat umfasst oder daraus besteht, zur Verwendung als ein Impfstoff in der Behandlung oder Vorbeugung von Blasenkrebs.

13. Verbindung zur Verwendung bei der Behandlung von Blasenkrebs nach Anspruch 12, wobei die Verbindung in einer Formulierung enthalten ist, die zur intramuskulären oder systemischen Verabreichung geeignet ist.

## Revendications

1. Méthode analytique pour détecter le cancer de la vessie, comprenant l'analyse d'un échantillon d'urine pour la présence du glycane constitué de Galβ1-4GlcNAcβ1-3Galβ1-4Glc (lacto-N-neo-tetraose, neolactotetra, nLc4).

2. Méthode analytique selon la revendication 1, **caractérisée par** l'analyse de la présence du glycane constitué de nLc4 par la mise en contact de l'échantillon avec un peptide ayant la spécificité de lier le nLc4.

3. Méthode analytique selon l'une des revendications précédentes, **caractérisée par** l'analyse de la présence du glycane constitué par nLc4 par électrophorèse sur gel capillaire des glycans obtenus par hydrolyse des glycosphingolipides d'une fraction de vésicule membranaire acellulaire isolée de l'échantillon d'urine.

4. Méthode analytique selon l'une des revendications précédentes, **caractérisée par le fait qu'**une concentration molaire d'au moins 1 fois l'étalon interne Oligomannose 6 (Man6) ou d'au moins 1 % de nLc4 du total des glycanes provenant des glycosphingolipides de l'échantillon indique la présence d'un cancer de la vessie.

5. Méthode analytique selon la revendication 4, **caractérisée par le fait que** la concentration molaire est déterminée par l'intensité du signal.

6. Méthode analytique selon l'une des revendications précédentes, **caractérisée par** l'analyse supplémentaire de l'échantillon d'urine pour la présence du glycane constitué de lactose.

7. Méthode analytique selon l'une des revendications précédentes, **caractérisée par** l'analyse supplémentaire de l'échantillon d'urine pour la présence du glycane constitué de GalNAcβ1-3Galα1-4Galβ1-4Glc (globotétraose, Gb4).

8. Méthode analytique selon l'une des revendications précédentes, **caractérisée par** l'analyse supplémentaire de l'échantillon d'urine pour la présence du glycane constitué de Galα1-4Galβ1-4Glc (globotriaose, Gb3).

9. Méthode analytique selon l'une des revendications précédentes, **caractérisée par** la préparation d'une fraction membranaire à partir de l'échantillon d'urine, à partir de laquelle est préparée une fraction lipidique, et par l'analyse de la fraction lipidique pour la présence du glycane.

10. Procédé analytique selon l'une des revendications précédentes, **caractérisé par le fait que** le résultat de l'analyse de l'échantillon pour la présence du glycane est transmis au patient dont provient l'échantillon ou à une personne agissant pour le compte du patient.

11. Anticorps ou CAR exprimé par un lymphocyte T, ou une partie de celui-ci, ayant la spécificité de lier un glycane constitué de nLc4, pour utilisation dans le traitement du cancer de la vessie.

12. Composé comprenant ou consistant en nLc4 et/ou nLc4-céramide et/ou un conjugué nLc4-protéine pour utilisation comme vaccin dans le traitement ou la prévention du cancer de la vessie.

13. Composé utilisé dans le traitement du cancer de la vessie selon la revendication 12, dans lequel le composé est compris dans une formulation adaptée à une administration intramusculaire ou systémique.
